Europäisches Patentamt

(19) **European Patent Office**

**Office européen des brevets**

(11) Publication number: **0 062 574**
A2

# EUROPEAN PATENT APPLICATION

(21) Application number: **82400564.9**

(22) Date of filing: **29.03.82**

(51) Int. Cl.³: **C 12 N 15/00,** C 12 N 7/00

(30) Priority: **31.03.81 US 249352**

(43) Date of publication of application: **13.10.82**
**Bulletin 82/41**

(84) Designated Contracting States: **AT BE CH DE FR GB IT
LI LU NL SE**

(71) Applicant: **THE REGENTS OF THE UNIVERSITY OF
CALIFORNIA, 485 University Hall, Berkeley,
California 94720 (US)**

(72) Inventor: **Rutter, William J., 80 Everson Street, San
Francisco California 94131 (US)**
Inventor: **Laub, Orgad, 201 Morningside Drive, San
Francisco California 94132 (US)**
Inventor: **Rail, Leslie B., 222 Dellbrook, San Francisco
California 94131 (US)**

(74) Representative: **Corre, Jacques Denis Paul et al, Cabinet
Regimbeau 26, Avenue Kléber, F-75116 Paris (FR)**

(54) **Virus protein synthesis.**

(57) The present invention provides expression of the S-antigen of HBV in eucaryotic host cells, especially mammalian cells, whereby post-translational processes sufficient for formation of S-antigen aggregates occur, under conditions of minimum risk of contamination. The S-antigen aggregates produced are highly antigenic, capable of eliciting protective immunity against HBV infection, hence suitable for incorporation into a vaccine. A significant contribution of the present invention is the use of the normal S-antigen promoter of HBV, either alone or in tandem with a second promoter. The invention further provides a vector for HBV core antigen expression using the HBV core antigen promoter. The use of HBV core antigen promoter permits construction of an expression vector independent of any preexisting promoter of the vector. The core antigen promoter is also very active and will be useful for the construction of vectors for expression of other proteins.

ACTORUM AG

# VIRUS PROTEIN SYNTHESIS

## BACKGROUND AND PRIOR ART

The operations of recombinant DNA technology include the isolation and purification of DNA coding for a particular desired protein, the transfer of the desired DNA to a different host organism, the expression of the transferred DNA by the host organism resulting in synthesis of the desired protein, and the isolation and purification of the desired protein from the host organism. Transfer is accomplished by inserting the desired DNA into a transfer vector which is DNA, either of plasmid or viral origin, capable of autonomous replication in the chosen host. To date the major emphasis has been on the use of procaryotic, e.g., bacterial, hosts and transfer vectors therefor. Bacterial hosts have the advantage of rapid growth rate on inexpensive media on a large scale. They have the disadvantage that they lack the inherent capability to carry out certain aspects of post-transcriptional and post-translational processing required for the accurate expression of many eucaryotic genes.

Eucaryotic genes frequently contain internal untranslated regions, termed introns, not found in procaryotes. Post-transcriptional processing of RNA transcribed from a eucaryotic gene is performed in the nucleus of a eucaryotic cell, in a series of cutting and splicing steps, resulting in messenger RNA with contiguous coding segments. Procaryotic cells lack the ability to carry out the necessary splicing reactions. As a consequence, many eucaryotic genes cannot be directly transferred and expressed in a procaryotic host. The prior art has resorted to the expedient of cloning cDNA, a reverse transcript of messenger RNA isolated from the eucaryotic donor cell. The cDNA method is limited to circumstances where mRNA coding for the desired protein is the predominant mRNA isolated from the donor cells or can be differentially isolated by special techniques. In many instances, however, the direct isolation of the corresponding eucaryotic gene is the only practical alternative. Such genomic clones must be expressed in a eucaryotic host cell.

An additional disadvantage of procaryotic hosts is presented by their inability to carry out certain post-translational processing steps. Such steps may include specific glycosylation and phosphorylation steps, the formation of specific disulfide crosslinks and the removal of certain peptides, for example. Eucaryotic host cells can normally carry out such steps, while the prior art reliance on procaryotic hosts necessitates resorting to in vitro chemical or enzymatic reactions which are difficult to control.

- 3 -                    00016

Despite these difficulties, the use of eucaryotic host cells is preferred where post-translational processing steps yield a more desirable product. For example, in the case of Hepatitis B virus (HBV), a major antigen, the surface (S) antigen, is a primary factor in eliciting protective immunity against the virus. The S-antigen is a component of the outer envelope of the virus. Plasma from persistently infected individuals also contains characteristic 22nm spherical particles which are aggregates of S-antigen monomers, lipid and other proteins. About half of the S-antigen molecules in the 22nm particles are glycosylated. Inasmuch as HBV cannot be grown in cultured cells or in laboratory animals, the only current source is from infected donor individuals. Purified 22nm particles have been shown to elicit protective immunity upon administration to non-immune volunteers. The lack of a convenient source of antigen particles limits their usefulness as a vaccine for large-scale immunization.

The entire genome of HBV has been cloned in E. coli, and its nucleotide sequence determined.* The amino acid sequences of the surface and core antigens have been inferred from the nucleotide sequences of HBV DNA. However, the nucleotide sequence data do not permit a prediction of the function of untranslated regions, or the existence of translated but subsequently processed regions, such as signal peptides. The location of a promoter sequence for a given eucaryotic gene is unpredictable; however, a

---

* See copending application Ser. No. 107,267, incorporated herein by reference.

promoter will frequently include a sequence resembling the "Hogness box", TATAA, 21 to 32 base pairs upstream from the start of the coding region. (See Corden, J., et al, Science, 209, 1406 (1980).) In the case of HBV S-antigen, such a region was not identified near the start of the region coding for mature S-antigen. Both monomeric S-antigen and the core antigen of HBV have been synthesized by bacteria containing the respective coding regions of HBV DNA on a suitable transfer vector. Expression by procaryotic hosts results in production of non-glycosylated, monomeric S-antigen either directly or as a fusion protein having a procaryotic $NH_2$-terminal portion. (See Application Ser. No. 107,267.) The procaryotic expression products are antigenic, but to a lesser extent than the 22nm particles. A desired goal would be the application of recombinant DNA techniques to achieve expression of S-antigen in a eucaryotic host, in which the post-translational processes yielding 22nm particles or other S-antigen aggregates could occur. Such expression has reportedly been achieved, in monkey cells, using an S-antigen coding region inserted into SV-40, expressed under control of the SV-40 late promoter.[*] At least some of the S-antigen expressed under these conditions was in the form of S-antigen aggregates visible in the electron microscope, indicating that the requisite post-translational processes could occur during expression in monkey cells. SV-40 can infect human cells and is capable of causing tumor transformation. Therefore, the SV-40 system is disadvantageous for the purpose

---

[*] Hamer, D., First International Congress for Recombinant DNA Research (1981).

of making a vaccine because of the risk of virus contamination of extracts of SV-40-infected cells.

## SUMMARY OF THE INVENTION

The present invention provides expression of the S-antigen of HBV in eucaryotic host cells, especially mammalian cells, whereby post-translational processes sufficient for formation of S-antigen aggregates occur, under conditions of minimum risk of contamination. The S-antigen aggregates produced are highly antigenic, capable of eliciting protective immunity against HBV infection, hence suitable for incorporation into a vaccine. A significant contribution of the present invention is the use of the normal S-antigen promoter of HBV, either alone or in tandem with a second promoter. Expression of S-antigen may thereby be achieved in a variety of vectors, independent of the existance or orientation of other promoters of the vector. The S-antigen promoter is highly active and should prove useful by itself, for enhancing the expression of other genes in eucaryotic host. As a further aspect of the invention, synthesis of S-antigen is enhanced by the use of the S-antigen promoter in tandem with, and in the same orientation as, a second promoter. The invention therefore provides a novel and unobvious expression vector for synthesis of S-antigen in eucaryotic host cells and for the production of S-antigen aggregates. The S-antigen aggregates provide a more highly antigenic form of S-antigen than that produced by bacterial hosts. The invention provides a vaccine comprising the S-antigen aggregates produced according to the invention, or using the expression vector of the invention.

The invention further provides a vector for HBV core antigen expression using the HBV core-antigen promoter. The use of HBV core antigen promoter permits construction of an expression vector independent of any pre-existing promoter of the vector. The core antigen promoter is also very active and will be useful for the construction of vectors for expression of other proteins.

DETAILED DESCRIPTION OF THE INVENTION

The starting point in the present invention is cloned HBV-DNA, carried on a plasmid transfer vector, such as pBR325, maintained and replicated in a strain of E. coli. Knowledge of the nucleotide sequence of the cloned HBV genome, which totals 3221 base pairs, allows for determining the relative location of the three major viral proteins, surface antigen, core protein and DNA polymerase, predicting their amino acid sequences and locating the sites of action of various restriction endonucleases.[*] The HBV DNA nucleotide sequence may also provide information as to the location of TATAA-like sequences usually associated at sites of initiation of transcription. However, to unequivocally locate and identify a promoter sequence, it is necessary to carry out in vitro experiments. The general strategy employed herein was to use HBV DNA, cut at various points by a restriction endonuclease, as a template for in vitro transcription. The greater the template length between the initiation point (promoter) and the restriction endonuclease cleavage site, the longer the RNA product. In order to provide a

_____

[*]   (as set forth in application Ser. No. 107,267)

0062574

meaningful reflection of eucaryotic transcription, an *in vivo* system extracted from eucaryotic cells should be employed. RNA polymerase II is the enzyme responsible for mRNA production in mammalian cells. The length of the RNA product can be measured by gel electrophoresis, which separates nucleic acids on the basis of their length. The amount of RNA synthesized can be determined by measuring the incorporation of a radio-labelled precursor of known specific activity into the RNA product.

Once the promoter has been identified, the next step is to construct an expression vector for expression of a coding region. Having demonstrated herein the activity of the HBV S-antigen and core protein promoters, it will be understood that these same promoters are useful for directing expression of other coding regions as well. Indeed, the high level of activity of the HBV promoters suggests their use in a wide variety of expression systems, where high levels of expression are desired.

An expression vector suitable for eucaryotic cells must be capable of replication in the host cell, in addition to providing a suitably located restriction site for inserting the promoter and coding region. Preferably, the vector itself should not be responsible for production of toxic substances, since it would be desirable to use the desired expression products for human administration, e.g., as a vaccine. However, the utility of HBV antigens is not limited to direct administration to produce active immunity. The antigens are also useful to produce corresponding antibodies in animals, and such antibodies are useful in diagnostic testing and for providing passive immunity. The antigens are also useful to produce monoclonal antibodies.

For the purpose of demonstrating HBV-S-antigen expression in a eucaryotic cell, an expression vector based on the virus SV-40 was employed. The advantageous features of SV-40 include the fact that it is a well-characterized virus; it has an active promoter, the "late" promoter, of its own; and its replication in the host cell provides an enormous gene amplification, up to 100,000 copies per cell late in the infection cycle.[*] As a system for demonstrating the practicality of the invention, SV-40 provides ease of manipulation, high levels of expression and a means for exploring the effects of promoters in tandem. For producing S-antigen for use in a vaccine, SV-40 has the disadvantage of being a virus capable of infecting human cells and of producing tumor transformation. It will be understood that other eucaryotic vector systems, for example adenovirus, are to be preferred where the expression product must be administered to human beings. Another suitable system employs COS cells bearing an integrated SV-40 genome and producing the large T antigen required for SV-40 replication (Gluzman, Y., Cell, 23, 175 (1981)). Such host cells are able to replicate any vector carrying an SV-40 replication origin region. Such vectors may be made defective in SV-40 late functions, by deletion or insertion of heterologous sequences. Such vectors are able to replicate in COS host cells, and to express the genes which they bear, without

---

[*] See, generally, Tooze, J. (1980) in DNA Tumor Viruses (J. Tooze, Ed.), cold Spring Harbor laboratory, Cold Spring Harbor, N.Y., part 2, pp. 175-204.

concomitant production of virus particles or infective viral DNA.

The SV-40 vectors disclosed herein demonstrate the effects of using an HBV promoter in tandem with and in opposition to the endogeneous SV-40 late promoter. Expression of HBV S-antigen and core protein were both demonstrated in host cells having the SV-40 vector with the late promoter oriented away from (opposite to) the HBV promoter (herein designated pESV-HVS⁻). Therefore, substantial expression was obtained from the HBV promoter alone. Some stimulation of expression was observed when the vector was constructed with the SV-40 and HBV promoters oriented in the same direction (in tandem), herein designated pESV-HVS⁺. Therefore the use of an HBV promoter in tandem with a vector promoter may enhance the yield of expression product.

The relationship of the HBV S-antigen promoter to the coding region of mature S-antigen is most unusual. The experimentally identified promoter lies 592 base pairs upstream from the codon for the $NH_2$-terminal amino acid of mature S-antigen, whereas TATAA-like sequences are usually found 21 to 32 base pairs upstream (Corden, J., et al, Science, 209, 1406 (1980)). If mature S-antigen is in fact the initial translation product, the large distance from the promoter to the start codon may have special significance in the control of S-antigen expression. However, a long region free of in-phase stop codons precedes the start of the region coding for mature S-antigen. Furthermore, a methionine start codon exists in this preceding region only 70 base pairs downstream from the S-antigen promoter. It is entirely likely, as an alternative, that the initial translation product from the S-antigen promoter is a much larger protein, comprising mature S-antigen and a pre-sequence, which may have an important function in the assembly of S-antigen aggregates such as the 22nm S-antigen particles.  In either case, the use of the normal S-antigen promoter in its normal relationship to the mature S-antigen coding region is considered to contribute to efficient expression of S-antigen aggregates, and may serve to explain the unexpected advantages of the invention.

As an alternative embodiment of the invention, the HBV region lying between the mature S-antigen and its promoter is deleted, effectively moving the promoter nearer the start codon of the mature S-antigen coding region.  At this point, the existence of introns in the deleted region cannot be ruled out.  Consequently, the alternative embodiment may be advantageous in certain vectors, e.g., yeasts, which do not carry out post-transcriptional processing.

Because of their high activity, the HBV promoters are useful for high rates of expression of a variety of proteins in eucaryotic hosts.  The present invention contemplates a eucaryotic expression vector comprising an HBV promoter.  The HBV promoter may be alone or placed in tandem with one or more additional eucaryotic promoters to modify the rate of expression of a coding region downstream therefrom.  Proteins such as HBV S-antigen and HBV core protein are synthesized by host cells containing the described vector.  Other proteins, especially proteins whose synthesis in mature form entails post-translational processing steps, are similarly synthesized using a vector of the present invention.

The following examples demonstrate identification of the HBV promoters, the construction of a suitable vector comprising an HBV promoter, synthesis of a protein under HBV promoter control, and production of a hepatitis vaccine comprising S-antigen synthesized under HBV promoter control in a eucaryotic host cell.

## EXAMPLE 1

### Identification of HBV promoters.

Transcription of HBV-DNA was carried out in an *in vitro* system, using cell-free extracts of HeLa S3 cells, as described by Manley, J. L., et al, *Proc. Nat. Acad. Sci. USA*, 77, 3855 (1980). DNA used as template in the reactions was derived from either plasmid pBam 1.85 or pEco 63. The former is pBR322 having the larger of two possible *BamHI* fragments of HBV inserted in the *BamHI* site, spanning the region from about 1.4 to 0.2 on the HBV map (see Figure 1). The latter is pBR325 having the entire HBV genome, cleaved at the *EcoRI* site, inserted in the *EcoRI* site of the plasmid. Construction of both plasmids is described in detail in copending application Ser. No. 107,267 incorporated herein by reference. Plasmid DNA was purified from extracts of transformed cells by standard methods, for example as described by Bolivar, R., et al, *Methods in Enzymology* (R. Wu, Ed.), Vol. 68, p. 245, Academic Press, New York, N.Y. (1979) and by Kahn, M., et al, *Ibid.*, p. 268. DNA of pBam 1.85 was further treated by digestion with a restriction endonuclease, as described *infra* and used without further purification in the transcription reaction. DNA of pEco 63 was digested with *EcoRI* endonuclease to release the full-sized HBV-DNA insert. The insert was separated by preparative gel electrophoresis and electroeluted from the gel, using standard techniques for example as described by Bolivar, F., supra, or by Southern, E., Ibid., p. 152.

To determine the start site of mRNA coding for the HBV core protein, the following DNA templates were tested: pBam 1.85 cleaved by HincII (at base 1685, according to the system of numbering HBV bases from the EcoRI site); pBam 1.85 cleaved by BstEII (at base 2824); pBam 1.85 cleaved by Bal I (at base 3010); and HBV-DNA cleaved by EcoRI (at base 1). (In comparing sequence numbers with those disclosed in copending application Serial No. 107,267, note that the starting point for numbering the bases was not the same. Thus, in the instant case, base number 1 corresponds to base number 1408 in application Serial No. 107,267). Approximate map locations can be seen by reference to Figure 1.

In the case of the core protein gene, a possible Hogness-box was identified at base 1654, approximately located by a triangle in Figure 1. Using the previously described restriction-endonuclease cut template DNA, transcription initiated near base 1654 would yield RNA products of the following expected sizes:

| | |
|---|---|
| HincII | 3 bases |
| BstEII | 1142 bases |
| BalI | 1328 bases |
| EcoRI | 1539 bases |

The in vitro transcription reactions were carried out as described by Manley, J. L., et al, supra. The reaction mixture contained [@-$^{32}$P] GTP,

( 400 Ci/£ mole) ATP, CTP and TTP, HeLa Cell S3 extract containing RNA polymerase II and HBV template DNA. After incubation for 60 minutes at 30°C, the RNA product was extracted from the reaction mixture with phenol/chloroform, ethanol precipitated, redissolved and anlyzed by electrophoresis on a 5% acrylamide-urea gel, as described by Maxam, A., et al, Methods in Enzymology, Vol, 65 (L. Grossman and K. Moldane, Eds.), p. 499. The gels resolved RNA molecules ranging in length from about 50 to 1500 bases. RNA transcripts were found to have the following sizes, using the indicated template DNA:

|        |                |
|--------|----------------|
| HincII | (not detected) |
| BstEII | 1100 bases     |
| BalI   | 1350 bases     |
| EcoRI  | 1600 bases     |

The results indicate initiation of mRNA synthesis near the Hogness box beginning at base 1654. The nucleotide sequence of HBV-DNA from base 1652 is TACATAAG.

The S-antigen promoter was determined experimentally by a similar series of in vitro transcription experiments. A possible Hogness box-sequence at base 2785 was noted, although it lies far upstream from the start of the mature S-antigen coding region. Template DNA used for these experiments was pBam 1.85 cleaved by BstEII endonuclease (base 2824), pBam 1.85 cleaved by BalI endonuclease (base 3010), pBam 1.85 cleaved by HincII endonuclease (base 3210), HBV-DNA cleaved by EcoRI (base 1) and pBam 1.85 cleaved by BamHI endonuclease (base 31). Transcription initiated near base 2785 would yield RNA products of the following expected sizes using template DNA cleaved by the indicated restriction endonuclease:

|        |           |
|--------|-----------|
| BstEII | 8 bases   |
| BalI   | 194 bases |
| HincII | 304 bases |
| EcoRI  | 405 bases |
| BamHI  | 436 bases |

In vitro reactions were carried out as described for the core protein. The resulting RNA transcripts were of the following observed sizes, using DNA cleaved by the indicated restriction endonuclease:

|        |              |
|--------|--------------|
| BstEII | Not Detected |
| BalI   | 200 bases    |
| HincII | 310 bases    |
| EcoRI  | 410 bases    |
| BamHI  | 440 bases    |

The data for BstEII cleaved DNA was confirmed using BstEII cleaved HBV-DNA purified from pEco 63. Additional RNA bands of 460 and 490 bases were observed with EcoRI and BamHI treated DNA, respectively. The results confirm the location of the S-antigen promoter near base 2785. The HBV-DNA nucleotide sequence from base 2783 is TATATAA. The actual initiation site of mRNA synthesis may be located about 60 bases upstream of that originally predicted, however, the location of the promoter is clearly identified by these experiments.

0062574

The amount of RNA formed under control of either HBV promoter was compared to the amount synthesized in the same system under control of the adenovirus late promoter, a known highly active promoter. The amount of $[\alpha\text{-}^{32}P]$-GTP incorporated into RNA was estimated by the density of autoradiograms of the gel electrophoresis plates. The HBV promoters resulted in comparable incorporation of radioactivity to the adenovirus promoter. Hence, the HBV promoters are themselves highly active in promoting synthesis of mRNA.

## EXAMPLE 2
### Construction of expression vectors
comprising an HBV promoter. In the following experiments, all restriction enzyme digestions were carried out under conditions recommended by the manufacturer, New England BioLabs, Beverly, Massachusetts. Units of restriction endonuclease activity are herein defined as an amount of enzyme needed to completely hydrolyze all susceptible sites in 1 µg of DNA in one hour at 37°C. For complete digestions, reaction mixtures contained 1 unit enzyme per µg DNA. For partial digestions with HpaI, 0.25 units/µg DNA was employed, and for partial digestion with BamHI, 0.5 units/µg DNA was employed. All batches of enzyme were individually assayed to calibrate their activity. Ligation reactions, catalyzed by DNA ligase, were carried out by standard methods, e.g., as described by Bolivar, F., et al, supra. The sequence of vector construction steps is diagrammed in Figure 2. Two vectors were constructed using the HBV S-antigen promoter, one having the HBV promoter in tandem with the SV-40 late promoter, the other having the two promoters in opposite orientation.  The former was designated pESV-HVS$^+$, the latter pESV-HVS$^-$.

For the purpose of both constructions, 20 μg pEco 63 was cleaved with BglII and HpaI endonuclease. A large fragment containing pBR325 and HBV sequences including S-antigen coding region was purified by preparative gel electrophoresis, essentially as described, supra.

For construction of pESV-HVS$^-$, 20 μg of SV-40 DNA was partially digested with HpaI endonuclease, then digested with BamHI endonuclease. The desired product, spanning from the HpaI site at 0.76 on the SV-40 map to the BamHI site at 0.14, was purified by preparative gel electrophoresis.

The resulting SV-40 and pEco 63 fragments were recombined in a DNA ligase-catalyzed reaction. The BamHI and BglII generated ends of the respective plasmids are single-stranded and self-complementary, hence cohesive, and join specifically together.  For a description of restriction endonucleases, see Roberts, R., Methods in Enzymology, Vol. 68 (R. Wu, Ed.), p. 27.  The HpaI-generated ends are blunt. Consequently the two fragments cannot self-ligate, and only recombine in one orientation with respect to each other.

The recombinant pEco 63/SV-40 plasmid was used to transform an ampicillin-sensitive E. coli strain by standard transformation techniques, e.g., as described by Bolivar, et al, supra.  Approximately 100 ampicillin-resistant colonies were obtained from ∼1 μg of recombinant pEco 63/SV-40 DNA.  The colonies were then tested by in situ hybridization, essentially as described by Grunstein, M., et al, Methods

0062574

in Enzymology, Vol. 68 (R. Wu, Ed.), p. 379, using
SV-40 DNA as a probe. Approximately 25 colonies
contained SV-40 sequences, and plasmid DNA was
isolated from several of these for further testing by
restriction endonuclease cleavage and gel electrophoresis to determine the presence of fragments of
predicted size and number. About 50% of these
isolates proved satisfactory.

A selected pEco 63-SV-40 recombinant plasmid
was purified and digested with EcoRI endonuclease to
remove the pBR325 DNA. The product was then
self-ligated. This step resulted in restoration of
an uninterrupted S-antigen coding region, as shown in
Figure 2. The HBV promoter and the SV-40 promoter
were in opposite orientation. The resulting vector
was designated pESV-HVS⁻.

The pESV-HVS⁻ vector was used to
cotransfect monkey CV-1 cells, together with DNA of a
replication-deficient SV-40 mutant.* Either a
thermosensitive mutant tsA58 or a deletion mutant
tsA$^\Delta$ was employed, the latter being preferred.
Productive infection could occur only in CV-1 cells
transfected with both DNAs (cotransfected).
Virus stocks were prepared from single plaque
isolates of cotransfected cells. The stocks were
mixtures of SV-40 (pESV-HVS⁻) and SV-40tsA$^\Delta$ (or
SV-40tsA58 depending on the helper virus employed).

The composition of the virus stocks was
verified by the Southern hybridization technique
(Southern, E. M., J. Mol. Biol., 98, 503 (1975)).

---

* Growth and transfection are described, e.g., by
Myers, R. M., et al, Proc. Nat. Acad. Sci. USA, 77,
6491 (1980).

Six days after co-infection with a virus stock prepared as described, free DNA was extracted from infected cells by the method of Hirt, B., _J. Mol. Biol._, 98, 503 (1967). The DNA was subjected to electrophoresis in a 1.4% agarose gel and transferred to nitrocellulose filters as described by Southern, E. M., _supra_. Hybridization to $^{32}$P-labelled HBV-DNA or SV-40 DNA revealed the positions of SV-40 and HBV DNA sequences after electrophoresis. With the SV-40 probe, two DNA bands were found, that of the tsA$^\Delta$ (or tsA58) helper and that of the recombinant ESV-HVS$^-$. The HBV-DNA probe hybridized exclusively to the recombinant DNA band. The experiment proved the existence of two distinct virus types in the stock, helper and recombinant.

Construction of pESV-HVS$^+$. For this construction, pEco 63 cleaved with _Bgl_II and _Hpa_I endonucleases, described _supra_, was employed. SV-40 DNA, 20 μg, was digested with _Hpa_II endonuclease to cleave the DNA at 0.72 on the SV-40 map. The linear DNA was then treated with S1 nuclease and DNA polymerase I to produce blunt ends, as described, for example, by Bolivar, F., et al, _supra_. The blunt ends were next attached to synthetic _Bam_HI oligonucleotide linkers (see Rothstein, R. J., et al, _Methods of Enzymology_, Vol. 68 (R. Wu, Ed.), p. 98), commercially available from Collaborative Research, Inc., Waltham, Massachusetts, by a standard technique, as described, e.g., by Goodman, H. M., et al, _Ibid._, p. 75. The linear DNA was then partially digested by _Hpa_I endonuclease, followed by _Bam_HI endonuclease digestion. The desired fragment of 2.8 kb, from the _Hpa_II site at 0.72 map units to the _Hpa_I site at 0.17 map units, was purified by gel electrophoresis and recombined with BglII/HpaI-cleaved pEco 63, as described, supra. Selection of ampicillin resistant transformants and characterization of the recombinant plasmids was carried out as described, supra. The pEco 63/SV-40 recombinant plasmid thereby obtained was treated with EcoRI endonuclease to remove the pBR325 sequences and to reconstitute the HBV S-antigen gene. The resulting vector, designated pESV-HVS$^+$, had the SV-40 late promoter oriented in tandem with the HBV S-antigen promoter, as diagrammed in Figure 2. Virus stocks comprising SV-40 (pESV-HVS$^+$) were propagated by cotransfection with helper DNA or by coinfection with helper virus, as described, supra.

Construction of vectors containing the HBV core protein promoter. The general strategy for construction of SV-40 vectors having tandem and opposite orientation of the SV-40 and core protein promoters is diagrammed in Figure 3. The techniques and procedures employed were essentially those already described.

SV-40 DNA was cleaved with restriction endonuclease HaeII at map unit 0.82. The linear DNA was treated with S1 nuclease and DNA polymerase I to provide blunt ends to which synthetic BamHI oligonucleotide linkers were attached by ligation. The DNA was then cleaved with BamHI endonuclease (at map unit 0.14) to produce a large fragment (spanning 0.14 to 0.82) purified by gel electrophoresis. The fragment was then inserted at the BamHI site of plasmid pBR322. The resulting recombinant was designated E-SV, and contained the origin of SV-40 replication and a functional A gene (required for SV-40 replication). A BamHI fragment, purified from pEco 63, was inserted into the BamHI site of pBR322. Two BamHI fragments were generated by cleavage of pEco 63. The larger fragment, containing the core protein gene and a short segment of pBR322, was purified by preparative gel electrophoresis before insertion into the BamHI site of pBR322. The resulting recombinant was designated E-HVC. The plasmids E-SV and E-HVC were prepared on a large scale ( 20 µg each). The plasmids were each treated with BamHI to release the SV-40 and HBV sequences, respectively. The respective sequences were then recombined with each other, in a DNA-ligase catalyzed reaction. The two possible orientations for the HBV sequence relative to the SV-40 sequence yielded two recombinants, ESV-HVC⁻ and ESV-HVC⁺, having the SV-40 promoter oriented in opposition and in tandem, respectively, with the HBV core protein promoter. Virus stocks were prepared and characterized as described, supra. ESV-HVC⁻ and ESV-HVC⁺ were distinguished by cleavage with restriction endonucleases having asymetrically located sites in the SV-40 and HBV sequences, so as to generate a unique set of restriction fragments from each vector recognizable by gel electrophoresis.

## EXAMPLE 3

### Expression of HBV genes in SV-40 vectors.

Synthesis of HBV mRNA was tested using all four SV-40-HBV recombinant viruses: SV-40 (pESV-HVS⁻), SV-40 (pESV-HVS⁺), SV-40 (pESV-HVC⁻) and SV-40 (pESV-HVC⁺). Monkey CV-1 cells were infected with one of the above viruses mixed with SV-40 tsA58 as helper. After six days at 40°C, cytoplasmic RNA was isolated, denatured and fractionated by electrophoresis on a 1.5% agarose methyl-mercury gel and transferred to diazotized benzyloxymethyl paper for hybridization to radio-labelled probe DNA, as described by Alwine, J. C., et al, Methods in Enzymology, Vol. 68 (R. Wu, Ed.), p. 220. The RNA was hybridized with nick-translated $^{32}$P-labelled HBV-DNA or SV-40 DNA, to reveal the position of SV-40 and HBV RNA's in the gel, by autoradiography. With the SV-40 probe, two RNA bands were visible, corresponding to 19S and 16S SV-40 mRNA. The HBV probe detected at least two mRNAs, one of which was the core gene transcript and one the S-antigen transcript. Synthesis of S-antigen and core protein is detected in monkey CV-1 cells infected with each one of the four recombinant viruses, labelled for six hours with $^{35}$S-cysteine. The S-antigen and core protein are immunoprecipitated from the cellular protein fraction and from the medium, with specific antibody to S-antigen and core protein, respectively, using formaldehyde-treated Staphylococcus aureus C to collect the antigen-antigody complexes, as described by Martial, J. A., et al, Proc. Nat. Acad. Sci. USA, 74, 1816 (1977). S-antigen is detected both in infected cells and in the medium. Significantly, S-antigen is detected in cells infected with SV-40 (ESV-HVS⁻), where the SV-40 promoter has opposite orientation from the HBV promoter. Therefore, expression of S-antigen is controlled entirely by the HBV S-antigen promoter in this instance. Core protein is detected primarily in the cellular protein fraction. Expression of core protein is observed in cells infected with SV-40 (ESV-HVC⁻) where the SV-40 promoter has opposite orientation from the HBV core protein promoter. Therefore, expression of core protein is controlled by the HBV core protein promoter in this instance.

Preparations of S-antigen partially purified from SV-40 (ESV-HSV⁻) infected cells are concentrated and prepared for electron microscopy (see Skelly, J., et al, *J. Gen. Virol.*, 44, 679 (1979)). Aggregates and particles resembling 22nm S-antigen particles are observed in the electron microscope field. Such aggregates are not observed from control cultures infected with helper virus only.

## EXAMPLE 4
### Preparation of a vaccine comprising S-antigen. Preferred vectors for synthesis of S-antigen for vaccine purposes are those which do not produce toxic or tumorigenic substances. Such vectors include adenoviruses, and various SV-40 systems that fail to make virus particles. For example, the recombinant vector pSV01, described by Myers, R. M., *Proc. Nat. Acad. Sci. USA*, 77, 6491 (1980), contains a 311 bp fragment including the SV-40 origin of DNA replication, the early promoter and the T antigen binding site, inserted in pBR322. The vector replicates in transfected monkey COS-1 cells which contain integrated SV-40 and express T-antigen (Gluzman, Y., *Cell*, 23, 175 (1981)). Recombinants of pSV01 and HBV-DNA, containing the S-antigen promoter and coding region, provide a suitable vector for synthesis of S-antigen in COS-1 cells.

S-antigen particles are purified from cell extracts by standard methods, for example as described by Skelly, J., et al, supra. Purified S-antigen aggregates and 22nm particles are dialyzed against physiological saline or phosphate-buffered saline and adjusted to 200 µg protein/ml final concentration. Guinea pigs are injected subcutaneously at 9, 14 and 56 day intervals with 1 ml of the S-antigen preparation. The serum of the test animals is sampled at 0, 28, 56 and 84 days and assayed for antibody titre against Dane particles or S-antigen partially purified from infectious serum. The radioimmunoassay of Hollinger, F., et al, J. Immunol., 107, 1099 (1971) is employed. The majority of animals exhibit antibodies cross-reactive with S-antigen 84 days after administration of the protein. Similar results are obtained upon injection of monkeys. Accordingly, the immunologically active protein constituents of HBV, expressed by a eucaryotic cell that has been transformed by an expression vector encoding said protein and the HBV promoter thereof, are capable of eliciting antibodies cross-reactive with known immunologically reactive components of HBV.

The described proteins have the advantage of being available in significantly larger quantities than S-antigen obtained from Dane particles or carrier serum. Furthermore, there is no danger of accidental infection since there is no intact virus in the S-antigen expression product. By contrast, viral proteins purified from serum always pose the danger of viral contamination.

### EXAMPLE 5

As shown in Example 4, protein coded by the HBV genome and synthesized by a eucaryotic cell is capable of eliciting antibodies cross-reactive with HBV S-antigen and core protein. It therefore follows that such antigens and antigen aggregates, when purified as described and administered in a physiologically acceptable medium, constitute a vaccine for protection against infection by the virus.

Sixteen chimpanzees are divided into three groups. Group A (6 animals) is inoculated intravenously with 1.0 ml of B.O.B. Hepatitis B virus; Group B (4 animals) is inoculated intravenously with 1.0 ml containing 500 µg of S-antigen, synthesized and purified as described in Example 3 or 4, in physiological saline; Group C (6 animals) is the control group and receives no inoculation. All chimpanzees in Group A have evidence of clinical hepatitis B (either antigenemia, enzyme elevations and/or antibody response) within forty weeks. None of the animals in Groups B or C show evidence of clinical hepatitis B infection over the same 40-week period. The chimpanzees of Group B are rendered immune to subsequent challenge when inoculated intravenously with 1.0 ml of B.O.B. Hepatitis B virus.

While the invention has been described in connection with specific embodiments thereof, it will

0062574

- 25 - 00016

be understood that it is capable of further modifications and this application is intended to cover any variations, uses or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

WHAT IS CLAIMED IS:

1. A DNA vector capable of replicating in a eucaryotic cell for expression in said cell of a protein coded by a DNA coding region, comprising said coding region and a promoter region of HBV S-antigen or core protein oriented in the same transcriptional direction as said coding region.

2. The DNA vector of Claim 1, having additionally a second promoter oriented in tandem with said HBV promoter.

3. The DNA vector of Claim 1, having additionally a second promoter in the opposite orientation from said HBV promoter.

4. The DNA vector of Claim 1, wherein the coding region comprises the region of the HBV genome coding for mature S-antigen, and the HBV promoter is the S-antigen promoter.

5. The DNA vector of Claim 1, wherein the coding region comprises the region of the HBV genome containing the HBV S-antigen promoter and the entire S-antigen coding region.

6. The DNA vector of Claim 4 or 5, comprising a segment of SV-40 DNA.

7. The DNA vector of Claim 4 or 5, comprising a segment of adenovirus DNA.

8.   The DNA vector of Claim 2 or 3, comprising the SV-40 late promoter.

9.   The DNA vector of Claim 5, comprising additionally the SV-40 late promoter oriented in tandem with the HBV promoter.

10.   The DNA vector of Claim 5, comprising additionally the SV-40 late promoter in the opposite orientation from the HBV promoter.

11.   The DNA vector of Claim 9, comprising SV-40 (ESV-HVS$^+$).

12.   The DNA vector of claim 10, comprising SV-40 (ESV-HVS$^-$).

13.   The DNA vector of Claim 1, wherein the coding region comprises the region of the HBV genome coding for core protein, and the HBV promoter is the core protein promoter.

14.   The DNA vector of Claim 13, comprising a segment of SV-40.

15.   The DNA vector of Claim 13, comprising a segment of adenovirus.

16.   The DNA vector of Claim 13, comprising additionally the SV-40 late promoter oriented in tandem with said core protein promoter.

17.  The DNA vector of Claim 13, comprising additionally the SV-40 late promoter in opposite orientation from the core protein promoter.

18.  The DNA vector of Claim 16, comprising SV-40 (ESV-HVC$^+$).

19.  The DNA vector of Claim 17, comprising SV-40 (ESV-HVC$^-$).

20.  A method of making HBV S-antigen in a eucaryotic cell comprising infecting or transfecting said cell with a DNA vector capable of replication in said cell, comprising the region coding for S-antigen and the S-antigen promoter region, in the same transcriptional orientation as the coding region, culturing said eucaryotic cell under conditions permitting replication of the DNA vector and isolating S-antigen from the medium and cellular protein fraction of said cells.

21.  The method of Claim 20, wherein the DNA vector is SV-40 (ESV-HVS$^+$) or SV-40 (ESV-HVS$^-$).

22.  The method of Claim 20, wherein the DNA vector comprises a segment of SV-40.

23.  The method of Claim 20, wherein the DNA vector comprises a segment of adenovirus.

24.  A method of making HBV core protein in a eucaryotic cell comprising infecting or transfecting said cell with a DNA vector capable of replication in said cell, comprising the region coding for core protein and the core protein promoter region, in the same transcriptional orientation as the coding region, culturing said eucaryotic cell under conditions permitting replication of the DNA vector and isolating core protein from the medium and cellular protein fraction of said cells.

25. The method of Claim 24, wherein the DNA vector is SV-40 (ESV-HVC$^+$) or SV-40 (ESV-HVC$^-$).

26. The method of Claim 24, wherein the DNA vector comprises a segment of SV-40.

27. The method of Claim 24, wherein the DNA vector comprises a segment of adenovirus.

28. A vaccine comprising HBV S-antigen made according to Claim 20 or 21 in a physiologically acceptable medium.

29. A vaccine comprising HBV S-antigen made according to Claim 22 or 23, in a physiologically acceptable medium.